# EUROPEAN PATENT SPECIFICATION

(11) **EP 2 161 033 B1**
(45) Date of publication and mention of the grant of the patent: **01.05.2013**
(21) Application number: 08015884.3
(22) Date of filing: 09.09.2008
(51) Int. Cl.: A61K 38/48, A61P 15/16, A61P 15/18

(54) **Botulinum toxin to introduce temporal infertiliy in a vertebrate (e.g. human)**
Botulinumtoxin zur Erzeugung der zeitweisen Unfruchtbarkeit in einem Wirbeltier (z.B. beim Menschen)
Toxine botulinique pour introduire une infertilité temporaire chez un vertébré (par exemple, un humain)

(43) Date of publication of application: 10.03.2010
(73) Proprietor: Grafe, Susanne, Dr., 60599 Frankfurt (DE); Heinen, Florian, Prof. Dr. med., 80805 München (DE)
(72) Inventor: Grafe, Susanne, Dr., 60599 Frankfurt (DE); Heinen, Florian, Prof. Dr. med., 80805 München (DE)
(74) Representative: Manitz, Finsterwald & Partner GbR

(56) References cited:
- WO-A-00/74703
- WO-A-02/34286
- WO-A-02/074327
- WO-A-2008/000490
- WO-A1-2007/143294
- BARRIE J. HODGSON ET AL: "Effects of Adrenergic Drugs Administered During Ovum Transport and Chemical Sympathectomy of the Oviduct on Fertility in Rabbits" BIOLOGY OF REPRODUCTION, vol. 13, no. 2, September 1975 (1975-09), pages 142-146, XP002511835

## Description

### Field of the Invention

The present invention relates a chemodenervating agent for use in inhibiting temporarily and reversibly the fertility of a vertebrate (e.g. human).

### Background of the Invention

### A. Chemodenervating agents

Chemodenervation refers to the use of an agent to prevent a nerve from stimulating its target tissue, e.g. a muscle, a gland or another nerve. Chemodenervation is for example performed with phenol, ethyl alcohol, or botulinum toxin. Chemodenervation is for example appropriate in patients with focal dystonia or localized spasticity in one or two large muscles or several small muscles. It may be used to alleviate symptoms such as muscle spasm and pain, and hyperreflexia. Chemodenervating agents capable of interfering with muscle innovation may also be called "muscle relaxant".

The term "muscle relaxant" is used herein to refer to at least two major therapeutic groups: neuromuscular blockers and spasmolytics. Neuromuscular blockers act by interfering with transmission at the neuromuscular end plate and have no direct CNS activity. They are often used during surgical procedures, in intensive care and emergency medicine to cause partly or complete paralysis or dose dependent paresis, respectively (i.e. are used as an modulator of muscle tonus). Spasmolytics, also known as "centrally-acting" muscle relaxants, are used to alleviate musculoskeletal pain and spasms and to reduce spasticity in a variety of neurological conditions. Neuromuscular blockers and spasmolytics are often grouped together as muscle relaxants. Both terms refer to distinct groups of agents.

Neuromuscular-blocking drugs block neuromuscular transmission at the neuromuscular junction, causing paralysis or paresis of the affected skeletal muscles. This is accomplished either by acting presynaptically via the inhibition of acetylcholine (ACh) synthesis or release, or by acting postsynaptically at the acetylcholine receptor. Example of drugs that act presynaptically are botulinum toxin, tetrodotoxin and tetanus toxin.

The term "chemodenervation" also encompasses all effects which directly or indirectly are induced by the chemodenervating agent, therefore also comprising upstream, downstream or long-term effects of said chemodenervating agent. Therefore presynaptic effects are also encompassed as well as postsynaptic effects, tissue effects and/or indirect effects via spinal or afferent neurons.

One chemodenervating agent, botulinum toxin, although being one of the most toxic compounds known to date, has in the past been used for the treatment of a large number of conditions and disorders, some of which are described in e.g. WO-A-2008/000490 (PCT/EP 2007/005754.) Furthermore, commercial forms of botulinum toxin type A based on the botulinum toxin A protein complex are available under the tradename Botox^{®} (Allergan Inc.) and under the tradename Dysport (Ipsen^{®} Ltd.), respectively. A pharmaceutical composition based on a higher purified toxin preparation and comprising the neurotoxic component of botulinum toxin type A free of complexing proteins in isolated form is commercially available in Germany from Merz Pharmaceuticals GmbH under the tradename Xeomin^{®}.

### B. Temporal infertility treatment of vertebrates

The reliable control of fertility in vertebrates, is of high economic interest and applicable in a wide area of topics ranging from livestock breeding to human birth control.

In view of an increasing human population with over 300 million couples worldwide not having continual access to effective means of contraception, temporal fertility control is a major issue. Fertility control of animals is also becoming a necessary commodity to restrict overpopulation of wild and feral species, to manage animals that might be reservoirs for zoonotic diseases transmissible to humans and livestock and in farm and companion animals to prevent unwanted pregnancies.

The methods known in the state-of-the-art for influencing female fertility, i.e. contraception, can be summarized as follows:

### B.1 Physical methods

Physical methods may work in a variety of ways, among them: physically preventing sperm from entering the female reproductive tract; hormonally preventing ovulation from occuring; making the woman's reproductive tract inhospitable to sperm; or surgically altering the female reproductive tract to induce sterility. Some methods use more than one mechanism. Physical methods vary in simplicity, convenience and efficacy.

Barrier methods place a physical impediment to the movement of sperm into the female reproductive tract. Cervical barriers are devices that are contained completely within the vagina. The contraceptive sponge has a depression to hold it in place over the cervix. The cervical cap is the smallest cervical barrier. Depending on the type of cap, it stays in place by suction to the cervix or to the vaginal walls. The diaphragm fits into place behind the woman's pubic bone and has a firm but flexible ring, which helps it press against the vaginal walls.

Spermicide may be placed in the vagina before intercourse and creates a chemical barrier. Spermicide may be used alone, or in combination with a physical barrier.

### B.2 Hormonal methods

There are variety of delivery methods for hormonal contraception.

Combinations of synthetic oestrogens and progestins (synthetic progestogens) are commonly used. These include the combined oral contraceptive pill ("The Pill"), the Patch, the contraceptive vaginal ring ("NuvaRing") or Lunelle, a monthly injection.

Other methods contain only a progestin (a synthetic progestogen). These include the progestin only pill (the POP or 'minipill'), the injectables Depo Provera (a depot formulation of medroxyprogesterone acetate given as an intramuscular injection every three months) and Noristerat (Norethindrone acetate given as an intramuscular injection every 8 weeks), and contraceptive implants. The progestin-only pill must be taken at more precisely remembered times each day than combined pills. The various progestin-only methods may cause irregular bleeding during use.

Another method is Ormeloxifene (Centchroman) which is a selective oestrogen receptor modulator, or SERM. It causes ovulation to occur asynchronously with the formation of the uterine lining, preventing implantation of a zygote. It has been widely available as a birth control method in India since the early 1990s, marketed under the trade name Saheli. Centchroman is legally available only in India.

### B.3 Intrauterine methods

These are contraceptive devices which are placed inside the uterus. They are usually shaped like a "T" - the arms of the T hold the device in place. There are two main types of intrauterine contraceptives: those that contain copper (which has a spermicidal effect), and those that release a progestogen (in the US the term progestin is used).

The terminology used for these devices differs in the United Kingdom and the United States. In the US, all devices which are placed in the uterus to prevent pregnancy are referred to as intra-uterine devices (IUDs) or intra-uterine contraceptive devices (IUCDs). In the UK, only copper-containing devices are called IUDs (or IUCDs), and hormonal intrauterine contraceptives are referred to with the term Intra-Uterine System (IUS).

### B.4 Sterilization

Surgical sterilization is available in the form of tubal ligation for women. In women, the process may be referred to as "tying the tubes," but the fallopian tubes may be tied, cut, clamped, or blocked. This serves to prevent sperm from joining the unfertilized egg. The non-surgical sterilization procedure, *Essure,* is an example of a procedure that blocks the tubes. Sterilization should be considered permanent.

### B.5 Methods in development

➢ Praneem is a polyherbal vaginal tablet being studied as a spermicide, and a microbicide active against HIV.
➢ BufferGel is a spermicidal gel being studied as a microbicide active against HIV.
➢ Duet is a disposable diaphragm in development that will be pre-filled with BufferGel. It is designed to deliver microbicide to both the cervix and vagina. Unlike currently available diaphragms, the Duet will be manufactured in only one size and will not require a prescription, fitting, or a visit to a doctor.
➢ The SILCS diaphragm is a silicone barrier which is still in clinical testing. It has a finger cup molded on one end for easy removal. Like the Duet, the SILCS is novel in that it will only be available in one size.
➢ A vaginal ring is being developed that releases both estrogen and progesterone, and is effective for over 12 months.
➢ Two types of progestogen-only vaginal rings are being developed. Progestogen-only products may be particularly useful for women who are breastfeeding. The rings may be used for four months at a time.
➢ A progesterone-only contraceptive is being developed that would be sprayed onto the skin once a day.
➢ Quinacrine sterilization and the Adiana procedure are two permanent methods of birth control being developed.

The methods known in the state-of-the-art for influencing male fertility can be summarized as follows:

### B.6 Hormone-therapies which influence the production of spermatogonia

This category comprises all methods which make use of different sex-hormone therapies to suppress the production of spermatogonias in an early stage. The problem of most of this approaches is the necessity of systemic application, which leads to side-effects like reduced libido and hormonal disbalance.

### B.7 Vaccines or other immunomodulatory substances which attack spermatic-proteins

These methods activate the individual immune system to attack proteins which are part of the spermatozoon and hence lead to its deactivation. These methods demand a laborious introduction of immune-stimulating factors and are only applicable for long term fertility control in mammals. Furthermore neither sufficient efficacy nor local antibody production nor reversibility of the process can be guaranteed, since memory-effects of the immune system can be triggered by this approaches.

### B.8 Direct influence of the spermatozoa with spermicides

This category comprises methods where the spermatozoon is attacked directly with chemical, biological or physical methods. All these very diverse methods target the spermatozoa with the intention to render them incapable of fertilizing the female egg (i.e. spematocides, spermatostatica, etc.). A general problem of these methods is that they have to be applied each time before the reproductive act takes place and some of them actually have to be applied to the female rather than to the male sexual organs.

### B.9 Physically blocking of the spermatic "flow"

This methods make use of substances/devices which either physically block the spermatic ducts or make use of condoms or similar devices, hence stopping the flow of the spermatozoa into the female sexual organs. Whereas the first type of methods comprises the problem that "reactivation", i.e. an reopening of the ducts, in most of the times can only be achieved by surgery, the second type of methods, (i.e. the use condoms etc.) needs to be carried out each time before the reproductive act takes place.

### B.10 In development: Adjudin-Treatment

Adjudin (1-(2,4-dichlorobenzyl)-1H-indazole-3-carbohydrazide) is an analogue of an old drug known as lonidamine and is said to induce reversible germ cell loss from the seminiferous epithelium by disrupting cell adhesion function between sertoli and germ cells. It weakens the adhesion between the sertoli cell and maturing sperm leading to a sloughing and loss of the latter. It is tested right now in clinical phase II for its ability to induce temporal infertility. However, when taken orally, the drug has very low bioavailability. The oral dose effective for contraception is so high that there have been causes of muscle atrophy and significant side effects in the liver.

The problems of the state-of-the-art-techniques, as it has been outlined above, are now - at least partly - overcome by this invention. The applicant has surprisingly found that the administration of a chemodenervating agent to e.g. testicular tissues creates a state of temporal infertility in males.

International patent application WO 02/34286 A1 discloses a contraceptive method comprising the step of intracranially administering a botulinum toxin to a patient, thereby reducing an intracranial secretion of a hormone required for gametogenesis.

International patent application WO 02/074327 A2 relates to an agent comprising a light chain component including a light chain or a fragment thereof of a botulinum toxin, a butyricum toxin, a tetani toxin or variants thereof, a translocation component comprising a heavy chain or a modified heavy chain of a botulinum toxin, a butyricum toxin, a tetani toxin or variants thereof, and a targeting component which selectively binds to a GnRH receptor.

International patent application WO 00/74703 A2 describes the use of *Clostridium botulinum* toxin of the types A, B, C, D, E, F or G or of a mixture of two or more of these toxins for the treatment of diseases of the nervous system or dystonia, respectively, or for a cosmetic treatment of hyperhidrosis or formation of wrinkles.

### Summary of the Invention

The present invention relates to a chemodenervating agent for the use of inhibiting temporarily and reversibly the fertility of a vertebrate, wherein the chemodenervating agent is administered to an ovarian, uterine, vaginal tissue or organ, epididymis or a testicular tissue, and wherein the chemodenervating agent is a botulinum toxin of serotype A, B, C, D, E, F or G.

The chemodenervating agent thereby interferes with the consistency, storage, release, transport, mobility and/or biological milieu of vertebrate females or males in reproductive cells, reproductive transport ways, reproductive organs, reproductive actions, reproductive cycles, thereby resulting in temporal, reversible, non-destructive, repeatable, local, multilocal or combined applicable, dose-dependent, controllable infertility.

According to the present invention the chemodenervating agent is used to inhibit temporarily and reversibly the fertility of a vertebrate. In one embodiment the vertebrate is a male or female. In a further embodiment the vertebrate male or female is a human or an vertebrate animal. In a further embodiment the vertebrate animal is a mammal, marsupial, reptile, amphibian, bird or fish.

In one embodiment said ovarian, uterine and/or vaginal tissue or organ is selected from the group comprising Graaf-Follicle, Fimbria ovarica, Bartholin's glands, cervix, Corpus luteum, Fallopian tubes, ovaries, Skene's gland, uterus, vaginal fornix, vagina or vulva.

In another embodiment the chemodenervating agent is administered to a testicular tissuewhich is a gland necessary for male fertility, a muscle of the male productive system, a spermatic duct, a blood vessel, the scrotum, the testis or a combination of two or more thereof. In yet a further embodiment the muscle of the male productive system is a cremasteric muscle, a smooth muscle of the ducts, a branching smooth muscle coursing through collagenous tissue of the tunica albuginea, a contractile testicular smooth muscles, a smooth muscle of the testicular capsule, a smooth muscle of a testicular duct, a musculus bulbocavernosus, a musculus ischiocavernosus, a muscle bulbospongiosus, or a combination of two or more thereof.

In another embodiment the chemodenervating agent is administered to the *epididymis.*

In one embodiment the chemodenervating agent prevents the transport of the reproductive cells. In another embodiment the chemodenervating agent prevents the secretion of a gland. In yet another embodiment said agent influences the intrinsic water-content of an organ or organ part. In yet another embodiment said agent prevents the differentiation of a reproductive cell. In yet another embodiment said agent prevents the survival of a reproductive cell. In yet another embodiment said agent prevents the release of hormones.

In one embodiment the chemodenervating agent is administered by injection, a dermal-patch a suppository, a cream, an ointment, a gel, a hydrogel, an oil, a biodegradable carrier, a microsphere, a liposome, a micelle, a dextrane microparticle, a polymeric matrix, a gelatine composition carrier, an absorbent or any combination thereof.

According to the present invention the chemodenervating agent is a botulinum toxin of serotype A, B, C, D, E, F or G. In yet a further embodiment said neurotoxin is the neurotoxic component of botulinum toxin, free of any complexing proteins. WO-A-2007 143 294 discloses lacal administration of a botulinum toxin to testicular tissue for alleviating testicular pain.

In comparison with other methods for temporal and reversible fertility control the invention has the following advantages:
- one treatment leads to a reliable infertility up to 12 months (depending on the dosage)
- the effect of the treatment is fully reversible
- the treatment does not include surgery
- the treatment overcomes the need for systemic medicaments. The treatment prevents a broad range of possible side effects like ranging from liver problems to enhanced cancer susceptibility.
- high knowledge and experience with botulinum toxin
- the treatment is broadly applicable in the vertebrate animal kingdom.

### Detailed Description of the Invention

It was surprisingly found by inventive effort, that the application of a chemodenervating agent can introduces temporal reversible infertility in a vertebrate.

### A. Definitions:

"Vertebrate" is defined herein any member of the subphylum vertebrata, chordates with backbones or spinal columns. Therefore the term "vertebrate" encompasses humans, mammals, marsupials, reptiles, birds, amphibians and fish.

The term "male" is defined herein as the sex of an organism, which produces small mobile gametes, called spermatozoa. Each spermatozoon can fuse with a larger female gamete or ovum, in the process of fertilization. A male cannot reproduce sexually without access to at least one ovum from a female. The definition "male vertebrate" also encompasses human males.

The term "female" is defined herein as the sex of an organism, or a part of an organism, which produces ova (egg cells). The ova are defined as the larger gametes in a heterogamous reproduction system, while the smaller, usually motile gamete, the spermatozoon is produced by the male. A female individual cannot reproduce sexually without access to the gametes of a male (an exception is parthenogenesis). The definition "female vertebrate" also encompasses human females.

The term "mammal" in this document is defined as any warm-blooded, vertebrate characterized by the presence of sweat glands, including milk producing glands, and by the presence of hair, three middle ear bones used in hearing, and a *neocortex* region in the brain. A male or female human, dog, cat, pig, cow, horse, donkey, sheep, goat and deer is therefore encompassed by this definition of a mammal.

The term "marsupial" is defined herein as a mammal in which the female typically has a pouch in which it rears its young through early infancy. They differ from placental mammals in their reproductive traits.

The term "reptile" is defined herein as any air-breathing, ectothermic vertebrate that has skin covered in scales as opposed to hair or feathers.

The term "bird" is defined herein as any bipedal, warm-blooded, vertebrate that lays eggs.

The term "amphibian" is defined herein as all living tetrapods (four-legged vertebrates) that do not have amniotic eggs, are ectothermic and generally spend part of their time on land.

The term "fish" is defined herein as aquatic vertebrates that are typically ectothermic, covered with scales, and equipped with two sets of paired fins and several unpaired fins.

The term "injection" is defined as any process, which allows the person skilled in the art to administer the active agent to the target site by penetrating the skin. An incomplete number of examples for "injections" are subcutaneous, intra-muscular, intra-venous, intra-thecal, intra-arterial, etc.

The term "cell" is defined herein as the structural and functional unit of all known living organisms, thereby being the smallest unit of an organism that is classified as living. This definition inter alia encompasses germ cells like the male sperm cells (gametes, gamteocytes), the female gametes like oocyte, ovocyte, or rarely oöcyte or any other germ cell involved in reproduction. This definition also encompasses any other body cell, like for example nerve cell, muscle cell, stem cell, etc.

The term "testicular tissues" as used herein refers to any tissue of the male reproductive tract, comprising muscles of the male productive system e.g. a cremasteric muscle, a smooth muscle of the ducts, a branching smooth muscle coursing through collagenous tissue of the tunica albuginea, a contractile testicular smooth muscles, a smooth muscle of the testicular capsule, a smooth muscle of a testicular duct, a musculus bulbocavernosus, a musculus ischiocavernosus, a muscle bulbospongiosus.spermatic duct, the *ductus ejaculatorii* the *urethra,* the *epididymis,* the *tunica albuginea,* the *vasa deferentia,* the scrotum, the testis, the dartos tunic, the intercrural fascia, the cremasteric fascia, the infundibuliform fascia, the parietal tunica vaginalis, the visceral tunica vaginalis, the tunica albuginea, the testis lobule, a testicular septum, a testicular mediastinum, a sertoli cell, a sinus of the epididymis, a spermatic vein, a ductus artery, an internal spermatic artery, an internal muscular tunic, a testicular cajal cell, an epididymis, a vas deferens, a seminal vesicle, an ejaculatory duct, a prostate gland, a cowper's gland, bulbus gland, a corpus cavernosum, a penis glans, a corpus spongiosum, a corpus cavernosum, a prostatic urethra, a pendulous urethra, a bulbous urethra, an external urethral sphincter or any gland necessary for fertility.

### B. Applicability of Temporal Infertility Introduction

This reversible and temporal control of fertility allows for a broad range of applications, of which exemplarily some are mentioned hereinunder:

### Application of the Chemodenervating Agent in Females:

In one embodiment said tissues or organs are female reproductive tissues and/or organs. In one embodiment said tissue or organ is selected from the group comprising Graaf-Follicle, Fimbria ovarica, Bartholin's glands, cervix, Corpus luteum, Fallopian tubes, ovaries, Skene's gland, uterus, vaginal fornix, vagina or vulva.

### Application of the Chemodenervating Agent in Males:

In another embodiment said tissues or organs are male reproductive tissues and/or organs. In one embodiment said tissue is chosen from the group comprising Cowper's glands, epididymis, penis, prepuce, prostate, scrotum, seminal vesicles or any other testicular tissue, e.g. the spermatic ducts.

In one embodiment the chemodenervating agent is injected into the tissue. In another embodiment the chemodenervating agent is administered by topical (e.g. epicutaneous, enema, vaginal), enteral (e.g. by catheter technique, by gastric feeding tube, duodenal feeding tube, gastrostomy, rectal, vaginal), parental by injection or infusion (e.g. intravenous, intraarterial, intramuscular, subcutaneous, intradermal, intraperitoneal), transdermal patches, ultrasound guided injection , transmucosal application and/or other technical means used in surgery.

One embodiment of this invention is the fertility control of humans. The invention enables human males to control their own fertility in a reliable, temporal and reversible way without the need of surgery (vasectomy), of taking systemic drugs or of permanent usage of physical contraceptives.

The breeding of domestic animals is another broad field of interest, were this invention can be applied. It allows for a fast, reliable and cheap method of rendering a big number of animals infertile within a short time period. In broad scale animal production processes high numbers of animals can be treated without the adverse effects of systemic drugs (e.g. hormones) or conventional castration or sterilization methods. In one embodiment this process is enhanced by the usage of multiple-injection devices.

This method allows both the introduction of long term infertility in live-stocks, e.g. valuable breeding animals. The invention enables now the breeder to keep herds of different gender together. Costly separation of animals, e.g. during transport, would not be necessary anymore. Still it is possible, if desired, to re-activate the fertility of certain animal again, by stopping giving injections. If, for example the breeder desires to allow just one male out of a herd to propagate, he can easily leave out this male from the treatment, thus allowing just this male to mate.

The agent of this invention also allows for the treatment of pets and companion animals. The temporal feature of the inventive method allows for precise offspring control of e.g. racehorses, thoroughbred dogs, etc.

In general, it is envisaged that the invention will lead to a simplified introduction of temporal infertility in vertebrates during animal transports, husbandry, production management and many more.

Depending on the site of application and dosis, the level of hormones can be influenced i.e. by disabling hormone producing glands and/or organs. In one embodiment it is therefore envisaged, that the odor and taste, and therefore the quality of e.g. male meat is enhanced by this "anti-hormone" treatment. In another embodiment the temporal reduction in hormone levels also allows to influence the temper of the treated animal, e.g. aggressive males could be calmed down during transportation via this method. In another embodiment said treatment which leads to altered hormone levels, could be combined with other contraceptive treatments known in the art.

### C. The Chemodenervating Agent for Introducing Infertility

Without being bound to any particular theory, chemodenervating agent can interfere in several ways with the fertility of vertebrates. Depending on the administration Chemodenervating agents interfere
➢ with the genesis and/or survival of the reproductive cells, e.g. by triggering death signals, interfering with the cell cycle or cell differentiation.
➢ with the storage of the reproductive cells e.g. by changing the localization of the reproductive cells or the secretion of surrounding tissue.
➢ with the water content of glandular secretion thereby altering the consistency (i.e. viscosity) of the secretion.
➢ with the transport by the reproductive cells, e.g. by interfering with glandular secretion, muscle peristaltic or movement of the ciliated epithelium.
➢ with the motility of the reproductive cells, e.g. by interfering with the motility of the flagellum or amoeba movements.
➢ with the biological milieu e.g. by altering the acetylcholine levels.

Depending on the desired effect, the artisan is able to choose the appropriate target and route of administration. For example if the interference with peristaltic movements of the reproductive tubes is envisaged, the artisan will be urged to inject the chemodenervating agent into or adjacent to the said reproductive tubes. If, in another embodiment, the artisan desires to interfere with the production and differentiation of the reproductive cells, he will understand to administer the chemodenervating agent to the sites of cell genesis. If, in yet another embodiment, the artisan desires to interfere with the secretion of a certain gland, he will understand to inject the chemodenervating agent to said gland and/or the neurons innervating said gland.

When interfering with glandular secretion, it is surprisingly found, that only the water content of the secretion is changed. Thus other parameters probably important for cell survival like salt-content, pH, peptide-levels etc. are not changed.

In another embodiment the application of the chemodenervating agent leads to an interference in water-content of the organ or organ-part itself. The term "interference" is defined herein as a change of the water-content of the organ or organ-part as compared to its normal water-content. Thus allowing for a localized interference with organ function.

In general this invention allows the induction of infertility in vertebrates, e.g. humans, in a temporal, reversible, non-destructive, repeatable, local, multilocal or combined applicable, dose-dependent, controllable manner.

In one embodiment the chemodenervating agent is injected into tissue and/or organ selected from the group comprising Graaf-Follicle, Fimbria ovarica, Bartholin's glands, cervix, Corpus luteum, Fallopian tubes, ovaries, Skene's gland, uterus, vaginal fornix, vagina or vulva. Thereby, depending on the site of application, leading to an interference with glandular secretion, nerve activation, muscle movement, biological milieu fabrication and/or reproductive cell motility.

In another embodiment the chemodenervating agent is applied to any testicular tissue (cf. definition above), resulting in temporal infertility. This induced infertility comprises an-ejaculation, malproduction of spermatogonia, retrograde ejaculation, and/or no production of secretion liquids necessary for semen transport. In another embodiment the chemodenervating agent is injected into tissues belonging to or being adjacent to the spermatic ducts, which causes temporal infertility. In one embodiment the transport of spermatozoa is impaired, hence leading to a temporal loss of fertility. In another embodiment the production, mobility and/or differentiation of spermatogonia itself is compromised. In a further embodiment the production of secretion liquids is affected. Therefore this invention combines the beneficial effects of a vasectomy, which is considered one of safest methods for fertility control, with the advantage of reversibility.

In one embodiment the injected tissue is a gland or in proximity to a gland, necessary for male fertility. In another embodiment the injected tissue is a muscle. In yet another embodiment the injected tissue is a smooth muscle. In yet another embodiment the smooth muscle is adjacent to and/or part of a spermatic duct. In another embodiment the injected muscle is a striated muscle. In yet another embodiment the injected tissue is any smooth and/or striated muscle and/or muscle cell of the male productive system comprising e.g. a cremasteric muscle, a smooth muscle of the ducts, a branching smooth muscle coursing through collagenous tissue of the tunica albuginea, a contractile testicular smooth muscles, a smooth muscle of the testicular capsule, a smooth muscle of a testicular duct, a musculus bulbocavernosus, a musculus ischiocavernosus, a muscle bulbospongiosus.

In another embodiment the injected tissue belongs to the spermatic duct. In yet another embodiment the injected tissue is the testis. In yet a further embodiment the injected tissue comprises the *epididymis*. In yet another embodiment the injected tissue is at least one seleted from tissues comprising a scrotum, a testis,

In one embodiment the administration of said chemodenervating agent is done by one or a combination of methods suitable for administration of said agent, for example by injection, solution for topical application, dermal-patch, a suppository, a cream, an ointment, a gel, a hydrogel, an oil, a biodegradable carrier, a microsphere, a liposome, a micelle, a dextrane microparticle, a polymeric matrix, a gelatine composition, an absorbent or any other suitable application. In another embodiment the chemodenervating agent is administered via the urethra and/or a testicular ductus. In one embodiment, the injection is performed with a multi-injection device.

In another preferred embodiment this invention also covers multiple injections of two or more within less than three months and split dosages. This allows for an adjustment of the ideal dosage depending on the male vertebrate to be treated.

In one embodiment, e.g. in cases of livestock management of farm animals, the animal will be locked into position by an animal holding device, i.e. similar to the ones used for animal castration, and the injection will be performed. To facilitate high throughput administration with injection devices, e.g. veterinary injection pistols, are envisioned. Furthermore, since the invention allows for multiple injections in one animal, one can adjust the administered dose simply by changing the number of injections. This allows also for high-throughput treatment of heterogenous animal groups. The before mentioned techniques may also be used in human application. In another even more preferred embodiment local anaesthesia can be applied before the injection process.

In another embodiment, e.g. for human fertility control, the treatment involves an automatic device, which allows the self-treatment. In one embodiment injection-devices as used for diabetes self-medication are envisaged. In another embodiment the application is a dermal-patch a suppository, a cream, an ointment, a gel, a hydrogel, an oil, a biodegradable carrier, a microsphere, a liposome, a micelle, a dextrane microparticle, a polymeric matrix, a gelatine composition carrier, an absorbent or any other composition suitable for self-treatment.

### D.1 The Chemodenervating Agent

According to the present invention, the chemodenervating agent is a botulinum toxin of the antigenically distinct serotypes A, B, C, D, E, F, or G. Wherever the botulinum toxin serotype A, B, C, D, E, F or G are mentioned, also known neurotoxic variants of the serotypes are encompassed, like serotypes A1, A2, etc. In one embodiment the botulinum toxin is botulinum toxin A.

The terms "botulinum toxin" or "botulinum toxins" as used throughout the present application, refer to the neurotoxic component devoid of any other clostridial proteins, but also to the "botulinum toxin complex": The term "botulinum toxin" is used herein in cases when no discrimination between the complex or the neurotoxic component is necessary or desired. "BoNT" or "NT" are common used abbreviations. The complex usually contains additional, so-called "non-toxic" proteins, which we will refer to as "complexing proteins" or "bacterial proteins".

A pharmaceutical composition comprising the neurotoxic component of botulinum toxin type A in isolated form is commercially available in Germany from Merz Pharmaceuticals GmbH under the trademark Xeomin^{®}. The production of the neurotoxic component of botulinum toxin type A and B are described, for example, in the international patent applications WO 00/74703 and WO 2006/133818.

The term "neurotoxic component" also includes functional homologs found in the other serotypes of *Clostridium* botulinum. In one embodiment of the present invention, the neurotoxic component is devoid of any other *C. botulinum* protein, in one embodiment also devoid of RNA, which might potentially be associated with the neurotoxic component. The neurotoxic component may be the single chain precursor protein of approximately 150kDa or the proteolytically processed neurotoxic component, comprising the light chain (L_{c}) of approximately 50kDa and the heavy chain (H_{c}) of approximately 100kDa, which may be linked by one or more disulfide bonds (for a review see e.g. Simpson LL, Ann Rev Pharmacol Toxicol. 2004; 44:167-93).

In another embodiment, also isoforms, homologs, orthologs and paralogs of Botulinum toxin are encompassed, which show at least 50%, at least 60%, at least 70%, at least 80%, at least 90% or up to 100% sequence identity. The sequence identity can be calculated by any algorithm suitable to yield reliable results, for example by using the FASTA algorithm (W.R. Pearson & D.J. Lipman PNAS (1988) 85:2444-2448).

Within this invention, all forms of botulinum toxin, in particular the various serotypes, the various complexes of the neurotoxic component of botulinum toxin and its complexing accompanying proteins and the neurotoxic component of these botulinum toxins are to be used.

In addition thereto, modified as well as recombinantly produced neurotoxic components of botulinum toxins including the respective mutations, deletions, etc. are also within the scope of the present invention. With respect to suitable mutants, reference is made for example to WO 2006/027207 A1, WO 2006/114308 A1 and EP07014785.5. Furthermore, within the present invention, mixtures of various serotypes (in the form the neurotoxic component or recombinant form or both forms thereof, e.g. mixtures of botulinum neurotoxins of types A and B) may be used. The present invention, however, also refers to neurotoxins which are chemically modified, e.g. by peylation, glycosylation, sulfatation, phosphorylation or any other modification, in particular of one or more surface or solvent exposed amino acid(s). Preferably said modified neurotoxin which is used according to the teaching of the present invention is capable of mediating uptake into the presynaptic nerve-cell and can block release of vesicular secretion for example by cleaving one or more proteins of the SNARE complex.

The complex of neurotoxic component and bacterial proteins is referred to as *"Clostridium botulinum* toxin complex" or *"botulinum* toxin complex". The molecular weight of this complex may vary from about 300,000 to about 900,000 Da. The complexing proteins are, for example, various hemagglutinins. The proteins of this toxin complex are not toxic themselves but provide stability to the neurotoxic component during passage through the gastrointestinal tract. Medicaments on the basis of the botulinum toxin complex of types A, B and C are commercially available, type A botulinum toxin from Ipsen (under the trademark Dysport^{®}) and from Allergan Inc. under the trademark Botox^{®}.

The neurotoxic subunit of this complex is referred herein as the "neurotoxic component" of the botulinum toxin complex. The neurotoxic component of the botulinum toxin complex is initially formed as a single polypeptide chain, having, in the case of serotype A, a molecular weight of approximately 150 kDa. In other serotypes, the neurotoxic component has been observed to vary between about 145 and about 170 kDa, depending on the bacterial source. A medicament on the basis of the neurotoxic component of Botulinum toxin type A is available from the applicant under the tradename Xeomin^{®} in Germany.

In accordance with the teaching of the present invention it is possible that the medicament contains no proteins found in the botulinum toxin complex other than the neurotoxic component. The precursor of the neurotoxic component may be cleaved or uncleaved, however, in one embodiment the precursor has been cleaved into the heavy and the light chain. As pointed out elsewhere herein, the polypeptides may be of wild-type sequence or may be modified at one or more residues. Modification comprises chemical modification e.g. by glycosylation, acetylation, acylation or the like, which may be beneficial e.g. to the uptake or stability of the polypeptide. The polypeptide chain of the neurotoxic component may, however, alternatively or additionally be modified by addition, substitution or deletion of one or more amino acid residues.

### D.2 The Pharmaceutical Composition

### D.2.1 The neurotoxic component

The neurotoxic component referred to herein above, may be part of a composition or a pharmaceutical composition. This pharmaceutical composition to be used herein may comprise botulinum toxin, e.g. in the form of neurotoxic component as the sole active component or may contain additional pharmaceutically active components e.g. a hyaluronic acid or a polyvinylpyrrolidone or a polyethleneglycol, such composition being optionally pH stabilized by a suitable pH buffer, in particular by a sodium acetate buffer, and/or a cryoprotectant polyalcohol.

In one embodiment, the neurotoxic component has a biological activity of 50 to 250 LD₅₀ units per ng neurotoxic component, as determined in a mouse LD₅₀ assay. In another embodiment, the neurotoxic component has a biological activity of about 150 LD₅₀ units per nanogram. Generally, the pharmaceutical composition of the present invention comprises neurotoxic component in a quantity of about 6 pg to about 30 ng.

A "pharmaceutical composition" is a formulation in which an active ingredient for use as a medicament or a diagnostic is contained or comprised. Such pharmaceutical composition may be suitable for diagnostic or therapeutic administration (i.e. by intramuscular or subcutaneous injection) to a human patient.

In one embodiment, said composition comprises the neurotoxic component of botulinum toxin type A. Said composition is a reconstituted solution of the neurotoxic component of botulinum toxin. In another embodiment the composition further comprises sucrose or human serum albumin or both, still another embodiment the ratio of human serum albumin to sucrose is about 1:5. In one embodiment, the composition is Xeomin^{®}. In another embodiment, said human serum albumin is recombinant human serum albumin. Alternatively, said composition is free of mammalian derived proteins such as human serum albumin. Any such solution may provide sufficient neurotoxin stability by replacing serum albumin with other non-proteinaceous stabilizers (infra).

With regard to the composition and dosing of the medicament on the basis of botulinum toxin, and in regard to the composition, dosing and frequency of administration of the medicament on the basis of the neurotoxic component of botulinum toxin, reference is made to WO-A-2008/000490 (PCT/EP2007/005754) ("High Frequency Application of Neurotoxic Component of Botulinum Toxin").

The pharmaceutical composition may be lyophilized or vacuum dried, reconstituted, or may prevail in solution. When reconstituted, in one embodiment the reconstituted solution is prepared adding sterile physiological saline (0.9% NaCl).

### D.2.2 Additional Components ("Excipients")

Such composition may comprise additional excipients. The term "excipient" refers to a substance present in a pharmaceutical composition other than the active pharmaceutical ingredient present in the pharmaceutical composition. An excipient can be a buffer, carrier, antiadherent, analgesic, binder, disintegrant, filler, diluent, preservative, vehicle, cyclodextrin and/or bulking agent such as albumin, gelatin, collagen, sodium chloride, preservative, cryoprotectant and/or stabilizer.

### D.2.3 pH-Buffers

A "pH buffer" refers to a chemical substance being capable to adjust the pH value of a composition, solution and the like to a certain value or to a certain pH range. In one embodiment this pH range can be between pH 5 to pH 8, in another embodiment pH 7 to pH 8, in yet another embodiment 7,2 to 7,6, and in yet a further embodiment a pH of 7,4. In another embodiment the pharmaceutical composition has a pH of between about 4 and 7.5 when reconstituted or upon injection, in yet antother embodiment about pH 6.8 and pH 7.6 and in a further embodiment between pH 7.4 and pH 7.6.

In one embodiment the composition also contains a 1-100 mM, in another embodiment 10 mM sodium acetate buffer.

The pH ranges given mentioned above are only typical examples and the actual pH may include any interval between the numerical values given above. Suitable buffers which are in accordance with the teaching of the present invention are e.g. sodium-phosphate buffer, sodium-acetate buffer, TRIS buffer or any buffer, which is suitable to buffer within the above pH-ranges.

### D.2.4 Stabilizers

"Stabilizing", "stabilizes" or "stabilization" means that the active ingredient, i.e., the neurotoxic component in a reconstituted or aqueous solution pharmaceutical composition has greater than about 20%, 30%, 40%, 50%, 60%, 70%, 80%, 90%, and up to about 100% of the toxicity that the biologically active neurotoxic component had prior to being incorporated into the pharmaceutical composition. For further examples of stabilized botulinum toxin solutions the Europeanpatents EP-A-1997509 (EP07010912.9) and (EP07020025.8) EP-A-2048156.

Examples of such stabilizers are gelatin or albumin, in one embodiment of human origin or obtained from a recombinant source. Proteins from non-human or non-animal sources are also included. The stabilizers may be modified by chemical means or by recombinant genetics. In one embodiment of the present invention, it is envisaged to use alcohols, e.g., inositol, mannitol, as cryoprotectant excipients to stabilize proteins during lyophilization.

In another embodiment of the present invention, the stabilizer may be a non proteinaceous stabilizing agent comprising a hyaluronic acid or a polyvinylpyrrolidone (Kollidon^{®}), hydroxyethyl starch, alginate or a polyethylene glycol or any combination thereof, such composition being optionally pH stabilized by a suitable pH buffer, in particular by a sodium acetate buffer, or a cryoprotectant or both. Said composition may comprise in addition to the mentioned stabilizers water and at least one polyalcohol, such as mannitol or sorbitol or mixtures thereof. It may also comprise mono-, di- or higher polysaccharides, such as glucose, sucrose or fructose. Such composition is considered to be a safer composition possessing remarkable stability.

The hyaluronic acid in the instant pharmaceutical composition is in one embodiment combined with the instant neurotoxic component in a quantity of 0.1 to 10 mg, especially 1 mg hyaluronic acid per ml in a 200 U/ml botulinum toxin solution.

The polyvinylpyrrolidone (Kollidon^{®}) when present in the instant composition, is combined with the instant neurotoxic component in such a quantity to provide a reconstituted solution comprising 10 to 500 mg, especially 100 mg polyvinylpyrrolidone per ml in a 200 U/ml neurotoxic component of botulinum toxin solution. In another embodiment reconstitution is carried out in up to 8 ml solution. This results in concentrations of down to 12.5 mg polyvinylpyrrolidone per ml in a 25 U/ml neurotoxic component solution.

The polyethyleneglycol in the instant pharmaceutical composition is in one embodiment combined with the instant neurotoxic component in a quantity of 10 to 500 mg, especially 100 mg polyethyleneglycol per ml in a 200 U/ml botulinum toxin solution. In another embodiment, the subject solution also contains a 1-100 mM, in yet another embodiment 10 mM sodium acetate buffer. This ratio is also applied in case of lower concentrations of down to 25 U/ml neurotoxic component solution.

The pharmaceutical composition for use in accordance with the present invention in one embodiment retains its potency substantially unchanged for six month, one year, two year, three year and/or four year periods when stored at a temperature between about - 20°C and +70 °C. Additionally, the indicated pharmaceutical compositions may have a potency or percent recovery of between about 20% and about 100% upon reconstitution.

### D.2.5 Cryoprotectants

"Cryoprotectant" refers to excipients which result in an active ingredient, i.e., a neurotoxic component in a reconstituted or aqueous solution pharmaceutical composition that has greater than about 20%, 30%, 40%, 50%, 60%, 70%, 80%, 90%, and up to about 100% of the toxicity that the biologically active neurotoxic component had prior to being freeze-dried in the pharmaceutical composition.

In another embodiment, the composition may contain a polyhydroxy compound, e.g. a polyalcohol as cryoprotectant. Examples of polyalcohols that might be used include, e.g., inositol, mannitol and other non-reducing alcohols. Some embodiments of the composition do not comprise a proteinaceous stabilizer, or do not contain trehalose or maltotriose or lactose or sucrose or related sugar or carbohydrate compounds which are sometimes used as cryoprotectants.

### D.2.6 Preservatives

The terms "preservative" and "preservatives" refer to a substance or a group of substances, respectively, which prevent the growth or survival of microorganisms, insects, bacteria or other contaminating organisms within said composition. Preservatives also prevent said composition from undesired chemical changes. Preservatives which can be used in the scope of this patent are all preservatives of the state of the art known to the skilled person. Examples of preservatives that might be used include, inter alia, e.g. benzylic alcohol, benzoic acid, benzalkonium chloride, calcium propionate, sodium nitrate, sodium nitrite, sulphites (sulfur dioxide, sodium bisulfite, potassium hydrogen sulfite, etc.), disodium EDTA, formaldehyde, glutaraldehyde, diatomaceous earth, ethanol, methyl chloroisothiazolinone, butylated hydroxyanisole and/or butylated hydroxytoluene.

### D.2.7 Analgesics

The term "analgesic" relates to analgesic drugs that act in various ways on the peripheral and central nervous systems and includes inter alia Paracetamol^{®} (acetaminophen), the nonsteroidal anti-inflammatory drugs (NSAIDs) such as the salicylates, narcotic drugs such as morphine, synthetic drugs with narcotic properties such as Tramadol^{®}, and various others. Also included is any compound with a local analgesic effect such as e.g. lidocaine, benzylic alcohol, benzoic acid and others.

In one embodiment the analgesic is part of the composition, in another embodiment, the analgesic is administered before, during or after the treatment with the chemodenervating agent.

### D.3 Administration

As indicated above, the pharmaceutical composition comprising the botulinum toxin is administered, in one embodiment several times, in an effective amount for introducing the desired infertility.

In doing so, the artisan is requested to judge the appropriate dose depending on the desired effect, the patient and the organ. It is obvious to the person skilled in the art, that a short period infertility induction in a small organ requires only small doses, whereas long-term infertility induction in large organs might require 10, 100, or 1000times higher dosages. Accordingly the treatment of an organ in a small dog would require much smaller doses than the treatment of the same organ in a racing horse.

Therefore, the following dosing-examples should only be seen as rough indications for typical dosage ranges. Normally dosages up to about 2000 units can be used, but in general should not exceed 1000 units. In one embodiment the range lies between about 1 to about 400 units for human adults.

While the above ranges relate to the maximum total doses, the dose range per tissue/organ is in one embodiment within 3 to 6 units/kg body weight (b.w.), for small tissues/organs 0,5-2 U/kg b.w., in another embodiment 0,1-1 U/kg b.w., in another embodiment 5 -20 U/kg b.w.

As to the frequency of dosing, the re-injection interval is in one embodiment greater than 3 months, e.g. 3 to 6 months. This is particularly true when applying medicaments on the basis of the botulinum toxin complex, where there exists an increased likelihood for the occurrence of antibodies.

When applying medicaments on the basis of the neurotoxic component of botulinum toxin free of complexing proteins, such as Xeomin^{®}, a more frequent dosing is also possible. Thus, the medicament may be re-administered in intervals of between 2 weeks and less than 3 months, sometimes also 3-6 months and sometimes even up to 12 months. In another embodiment also intervals of less then 2 weeks, e.g. days, hours or even minutes are encompassed. In yet another embodiment the medicament is re-administered at a point in time when additional infertility induction is needed.

With regard to the composition and dosing of the medicament on the basis of botulinum toxin, and in regard to the composition, dosing and frequency of administration of the medicament on the basis of the neurotoxic component of botulinum toxin, reference is made to WO-A-2008/000490 (PCT/EP2007/005754) ("High Frequency Application of Neurotoxic Component of Botulinum Toxin").

In one embodiment said composition comprises only Botulinum toxin as an active component, in another embodiment further active components e.g. analgesics, said muscle activating agent, etc. are part of the composition.

While the above stated values are to be understood as a general guideline for administering the medicament as used within the present invention, it is, however, ultimately the physician who is responsible for the treatment who decides on both the quantity of toxin administered and the frequency of its administration. In the present method, the medicament on the basis of botulinum toxin is in one embodiment re-administered at a point in time at which the fertility of the patient is (again) recovering, e.g. the transport of spermatozoa is again detectable.

The medicament on the basis of botulinum toxin can be injected directly into or adjacent to the desired target-tissue and/or -organ. In order to find the appropriate injection site, several means exist which help the physician in order to find the same. Within the present invention, all methods for finding the best injection site are applicable, such as injection guided by electromyography (EMG), injection guided by palpation, injection guided by CT/MRI, as well as injection guided by ultrasound (ultrasonography). Among those methods, the latter is in one embodiment the method of choice.

### D.5 Further Definitions

The term "lyophilization" is used in this document for a treatment of a solution containing the chemodenervating agent, e.g. the neurotoxic component of the botulinum toxin, whereas this solution is frozen and dried until only the solid components of the composition are left over. The freeze-dried product of this treatment is therefore defined in this document as "lyophilisate".

The term "reconstitution" is defined as the process of solubilization of said freeze-dried composition of the chemodenervating agent, e.g. the neurotoxic component. This can be done by adding the appropriate amount of sterile water, e.g. if all necessary components are already contained in the lyophilisate. Or, if this is not the case, it can be done e.g. by adding a sterile saline-solution alone or if applicable with the addition of components comprising e.g. a pH buffer, excipient, cryoprotectant, preservative, analgesic stabilizer or any combination thereof. The saline of before mentioned "saline-solution" is a salt-solution, in another embodiment being a sodium-chloride (NaCl) solution, in yet another embodiment being an isotonic sodium-chloride solution (i.e. a sodium-chloride concentration of 0,9%). The solubilization is carried out in such a manner that the final "reconstitution" is directly or indirectly, i.e. for example after dilution, administrable to the patient. The neurotoxin may be reconstituted in isotonic media, e.g. in isotonic saline or in sterile saline.

### E Remarks

It must be noted that, as used in the specification and the appended claims, the singular forms "a", "an" and "the" include plural referents unless the context clearly dictates otherwise.

The term "about" refers to an interval around the considered value. As used in this patent application, "about X" means an interval from X minus 10% of X to X plus 10% of X, and preferably an interval from X minus 5% of X to X plus 5% of X.

Unless they are defined differently, all the technical and scientific terms used here have the same meaning as that usually understood by an ordinary specialist in the field to which this invention belongs.

Due to the findings on which the present invention is based, it is now possible to provide a chemodenervating agent as outlined above for the preparation of a medicament to introduce temporal and reversible infertility in a vertebrate (e.g. human).

## Claims

1. A chemodenervating agent for use in inhibiting temporarily and reversibly the fertility of a vertebrate, wherein the chemodenervating agent is administered to an ovarian, uterine, vaginal tissue or organ, epididymis or a testicular tissue, and wherein the chemodenervating agent is a botulinum toxin of serotype A, B, C, D, E, F or G.

2. The chemodenervating agent for use according to claim 1, wherein the vertebrate is a male or female.

3. The chemodenervating agent for use according to claim 1 or 2, wherein the vertebrate is a human or a vertebrate animal.

4. The chemodenervating agent for use according to claim 3, wherein the vertebrate animal is a mammal, marsupial, reptile, amphibian, bird or fish.

5. The chemodenervating agent for use according to any of the preceding claims, wherein said ovarian, uterine and/or vaginal tissue or organ is selected from the group comprising Graaf-Follicle, Fimbria ovarica, Bartholin's glands, cervix, Corpus luteum, Fallopian tubes, ovaries, Skene's gland, uterus, vaginal fornix, vagina or vulva.

6. The chemodenervating agent for use according to any of the claims 1 to 4, wherein the testicular tissue is a gland and/or a muscle of the male productive system, a spermatic duct, a blood vessel, the scrotum, the testis or a combination of two or more thereof.

7. The chemodenervating agent for use according to any of the preceding claims, wherein said agent is administered by injection, a dermal-patch, a suppository, a cream, an ointment, a gel, a hydrogel, an oil, a biodegradable carrier, a microsphere, a liposome, a micelle, a dextrane microparticle, a polymeric matrix, a gelatine composition carrier, an absorbent or any combination thereof.

8. The chemodenervating agent for use according to any of the preceding claims, wherein said agent is the neurotoxic component of botulinum toxin, free of any complexing proteins.

## Patentansprüche

1. Chemodenervierendes Mittel zur Verwendung für die temporäre und reversible Inhibition der Fertilität eines Wirbeltiers, wobei das chemodenervierende Mittel einem Ovarium, einer Gebärmutter, einem Vaginalgewebe oder -organ, Nebenhoden oder einem Hodengewebe verabreicht wird, und, wobei das chemodenervierende Mittel ein Botulinumtoxin vom Serotyp A, B, C, D, E, F oder G ist.

2. Chemodenervierendes Mittel zur Verwendung nach Anspruch 1, wobei das Wirbeltier ein Männchen oder Weibchen ist.

3. Chemodenervierendes Mittel zur Verwendung nach Anspruch 1 oder 2, wobei das Wirbeltier ein Mensch oder ein tierisches Wirbeltier ist.

4. Chemodenervierendes Mittel zur Verwendung nach Anspruch 3, wobei das tierische Wirbeltier ein Säugetier, ein Beuteltier, ein Reptil, eine Amphibie, ein Vogel oder ein Fisch ist.

5. Chemodenervierendes Mittel zur Verwendung nach einem der vorstehenden Ansprüche, wobei die Ovarie, Gebärmutter und/oder das Vaginalgewebe oder -organ aus der Gruppe ausgewählt ist, welche Graaf-Follikel, Fimbria ovarica, Bartholin-Drüsen, Cervix, Corpus luteum, Eileiter, Ovarien, Skene-Drüse, Gebärmutter, Vaginalfornix, Vagina oder Vulva umfasst.

6. Chemodenervierendes Mittel zur Verwendung nach einem der Ansprüche 1 bis 4, wobei das Hodengewebe eine Drüse und/oder ein Muskel des männlichen Reproduktionssystems, ein Spermienleiter, ein Blutgefäß, das Scrotum, der Hoden oder eine Kombination aus zwei oder mehr hiervon ist.

7. Chemodenervierendes Mittel zur Verwendung nach einem der vorstehenden Ansprüche, wobei das Mittel durch Injektion, ein Hautpflaster, eine Suppositorie, eine Creme, eine Salbe, ein Gel, ein Hydrogel, ein Öl, einen bioabbaubaren Träger, eine Mikrokugel, ein Liposom, eine Micelle, ein Dextran-Mikropartikel, eine Polymermatrix, einen Träger aus einer Gelatinezusammensetzung, ein Absorptionsmittel oder eine Mischung hiervon verabreicht wird.

8. Chemodenervierendes Mittel zur Verwendung nach einem der vorstehenden Ansprüche, wobei das Mittel die neurotoxische Komponente von Botulinumtoxin frei von irgendeinem komplexierenden Protein ist.

## Revendications

1. Agent de chimio-dénervation pour une utilisation dans l'inhibition temporaire et réversible de la fécondité d'un vertébré, ledit agent de chimio-dénervation étant administré à un tissu ou organe ovarien, utérin ou vaginal, à l'épididyme ou à un tissu testiculaire, et ledit agent de chimio-dénervation étant une toxine botulinique de sérotype A, B, C, D, E, F ou G.

2. Agent de chimio-dénervation pour une utilisation suivant la revendication 1, le vertébré étant un vertébré mâle ou femelle.

3. Agent de chimio-dénervation pour une utilisation suivant la revendication 1 ou 2, le vertébré étant un être humain ou un animal vertébré.

4. Agent de chimio-dénervation pour une utilisation suivant la revendication 3, l'animal vertébré étant un mammifère, un marsupial, un reptile, un amphibien, un oiseau ou un poisson.

5. Agent de chimio-dénervation pour une utilisation suivant l'une quelconque des revendications précédentes, ledit tissu ou organe ovarien, utérin et/ou vaginal étant choisi dans le groupe consistant en le follicule de De Graaf, la frange ovarienne, les glandes de Bartholin, le col de l'utérus, le corps jaune, les trompes de Fallope, les ovaires, la glande de Skene, l'utérus, le cul-de-sac vaginal, le vagin ou la vulve.

6. Agent de chimio-dénervation pour une utilisation suivant l'une quelconque des revendications 1 à 4, le tissu testiculaire étant une glande et/ou un muscle du système producteur mâle, un canal déférent, un vaisseau sanguin, le scrotum, les testicules, ou une association de deux ou plus de deux de ces tissus.

7. Agent de chimio-dénervation pour une utilisation suivant l'une quelconque des revendications précédentes, ledit agent étant administré par injection, au moyen d'un timbre dermique, d'un suppositoire, d'une crème, d'une pommade, d'un gel, d'un hydrogel, d'une huile, d'un support biodégradable, d'une microsphère, d'un liposome, d'une micelle, d'une microparticule de dextrane, d'une matrice polymère, d'un support ayant une composition à base de gélatine, d'un absorbant ou de n'importe laquelle de leurs associations.

8. Agent de chimio-dénervation pour une utilisation suivant l'une quelconque des revendications précédentes, ledit agent étant le constituant neurotoxique de la toxine botulinique, dépourvu de toutes protéines complexantes.
